# FASCICULE DE BREVET EUROPEEN

(11) **EP 3 792 813 B1**
(45) Date de publication et mention de la délivrance du brevet: **23.11.2022**
(21) Numéro de dépôt: 20205599.2
(22) Date de dépôt: 24.10.2016
(51) Int. Cl.: G06K 7/10, G16H 10/40

(54) **PROCÉDÉ DE MISE EN OEUVRE D'UN INVENTAIRE D'UNE PLURALITÉ DE CONTENEURS BIOLOGIQUES ET PORTIQUE ASSOCIÉ**
VERFAHREN ZUR DURCHFÜHRUNG EINER BESTANDSAUFNAHME EINER VIELZAHL VON BIOLOGISCHEN BEHÄLTERN, UND ZUGEHÖROGER PORTALKRANK
METHOD FOR IMPLEMENTING AN INVENTORY OF A PLURALITY OF BIOLOGICAL CONTAINERS AND ASSOCIATED GANTRY

(30) Priorité: 01.07.2016 FR 1656321
(43) Date de publication de la demande: 17.03.2021
(62) Demande divisionnaire de: 16785472.8
(73) Titulaire: Biolog-id, 92100 Boulogne-Billancourt (FR)
(72) Inventeur: MONGRENIER, Jean-Claude, 13100 Aix en Provence (FR)
(74) Mandataire: Lavoix

(56) Documents cités:
- EP-A1- 0 598 624
- EP-A1- 1 693 807
- FR-A1- 2 988 936
- US-A1- 2008 203 160

## Description

La présente invention concerne un procédé de mise en œuvre d'un inventaire d'une pluralité de conteneurs de produits biologiques et un appareil comprenant un portique RFID pour la mise en œuvre d'un inventaire d'une pluralité de conteneurs de produits biologiques.

La présente invention se rapporte au domaine de la logistique de conteneurs.

De tels conteneurs sont par exemple des poches contenant des produits biologiques tels que des produits sanguins (poches de sang primaire, de plasma, de plaquette, de globules rouges...) ou des produits d'ingénierie cellulaire (cellules souches...), ou encore des poches de médicaments telles que des poches de chimiothérapie.

Tout au long du transport des conteneurs, il est impératif d'assurer une exposition minimale des conteneurs à une température relativement élevée. Ainsi, pour certains produits biologiques, la législation impose un délai maximal d'exposition des conteneurs à une température supérieure à - 4°C. Ce délai maximal est fixé en Europe à 2 heures 30 par la directive.

Par ailleurs, dans un impératif de rentabilité, il convient de traiter un nombre important de conteneurs simultanément.

Dans le cas des poches de produits sanguins, il est connu que les poches sont mises dans un camion frigorifique sous forme de colis, ces colis étant parfois rassemblés pour former des ensembles aisés à décharger. L'opérateur décharge alors chaque ensemble dans une armoire en grille métallique ou un caddie à roulettes ou une palette portée par un transpalette que l'opérateur pousse jusqu'à une chambre intermédiaire à une température de 4°C. Dans cette chambre intermédiaire, l'opérateur vérifie la présence de chaque poche ou chaque ensemble de poches pour effectuer un inventaire par un scan des codes-barres présents sur les poches ou sur chaque ensemble de poches. Puis un tri est effectué, par exemple, selon la provenance ou la catégorie du produit sanguin. Chaque ensemble de poches est ensuite rechargé sur une palette. L'opérateur met ensuite les poches dans une chambre froide à environ - 40°C dans le cas de plasma durant une période dite de quarantaine, la période de quarantaine étant supérieure ou égale à 60 jours. Le plasma est alors apte à être fractionné.

Toutefois, le temps mis par l'opérateur pour un ensemble est relativement long, de l'ordre de 4 heures à 5 heures, et il convient de réduire ce temps pour respecter les exigences légales ou les bonnes pratiques afin d'assurer une certaine qualité.

Il existe donc un besoin pour un procédé de mise en œuvre d'un inventaire d'une pluralité de conteneurs de produits biologiques qui soit de mise en œuvre plus rapide.

Le document EP 0 598 624 A1 décrit un système d'identification comprenant un interrogateur et une pluralité de transpondeurs. L'interrogateur comprend un transmetteur de signaux aux transpondeurs et un récepteur pour recevoir des signaux de réponse des transpondeurs comprenant des données les identifiant.

Selon l'invention, ce but est atteint par un procédé de mise en œuvre d'un inventaire d'une pluralité de conteneurs de produits biologiques selon la revendication 1.

Suivant des modes de réalisation particuliers, le procédé comprend une ou plusieurs des caractéristiques selon les revendications 2 à 6 ou la caractéristique suivante, prise(s) isolément ou suivant toutes les combinaisons techniquement possibles :
- le réceptacle est à une distance d'un des panneaux de lecture inférieure à 10 cm lors du déplacement.

L'invention concerne aussi un appareil de mise en œuvre d'un inventaire d'un inventaire d'une pluralité de conteneurs de produits biologiques selon la revendication 7.

D'autres caractéristiques et avantages de l'invention apparaîtront à la lecture de la description qui suit de modes de réalisation de l'invention, donnée à titre d'exemple uniquement et en référence aux dessins qui sont :
- figure 1, une vue en perspective d'une palette comportant des conteneurs de produits biologiques sur laquelle un inventaire est à mettre en œuvre et un appareil de mise en œuvre d'un inventaire, l'appareil comportant un portique,
- figure 2, une représentation du portique de la figure 1 vue le long de la direction de traversée du portique,
- figure 3, une représentation du portique de la figure 1 vue le long d'une direction perpendiculaire à la direction de traversée du portique, et
- figure 4, une représentation d'un portique, vue le long d'une direction perpendiculaire à la direction de traversée du portique, selon une alternative non couverte.

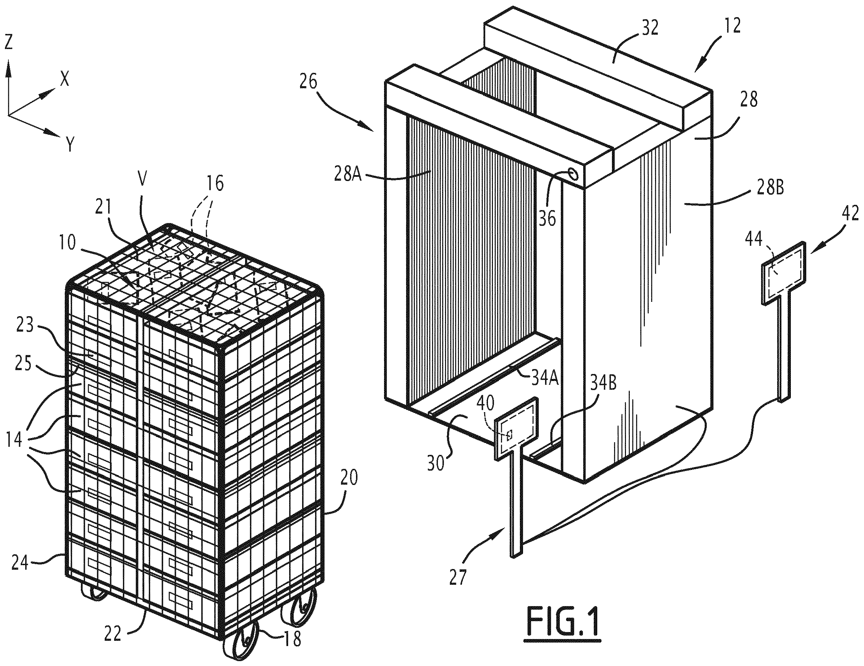

Un réceptacle 10 et un appareil 12 de mise en œuvre d'un inventaire sont représentés sur la figure 1.

Un réceptacle est un ensemble de transport, souvent désigné par le terme générique anglais de « roll ».

Le réceptacle 10 est destiné au transport de colis 14 d'un endroit à un autre.

Le réceptacle 10 est propre à accueillir un ensemble de colis 14.

Le nombre de colis 14 que le réceptacle 10 est propre à accueillir est supérieur à 22 colis.

Selon un exemple usuel, le nombre de colis 14 que le réceptacle 10 est propre à accueillir est égal à 22.

A titre d'illustration, seize colis 14 sont représentés sur la figure 1.

Chaque colis 14 présente la forme d'une boîte parallélépipédique.

Chaque colis 14 est réalisé en carton ou plastique.

Chaque colis 14 est propre à accueillir une pluralité de conteneurs 16, chaque conteneur 16 comportant des produits biologiques.

De manière générale, le conteneur 16 est tout type de poche destinée à contenir des produits dont l'utilisation est conditionnée par des contraintes de stockage strictes.

Dans un mode de réalisation, les conteneurs 16 sont des poches contenant des produits biologiques tels que des produits sanguins (poches de sang primaire, de plasma, de plaquettes, de globules rouges...) ou des produits d'ingénierie cellulaire (cellules humaines ou animales, notamment cellules souches humaines ou animales, produits issus de cellules humaines ou animales).

Dans un autre mode de réalisation, les conteneurs 16 sont des poches de médicaments ou préparations thérapeutiques contenant un ou plusieurs principes actifs ou médicaments, telles que des poches de chimiothérapie (contenant généralement un soluté et un ou plusieurs principes actifs de chimiothérapie).

De manière plus générale, le conteneur 16 est propre à contenir tout produit destiné à être perfusé à un patient (humain ou animal).

Selon l'exemple considéré, chaque conteneur 16 est une poche destinée, dans le cas présent, à contenir du plasma.

De façon connue, un tel conteneur 16 est un contenant étanche de plasma en matière plastique respirante permettant le métabolisme, du type PVC (polychlorure de vinyle), polycarbonate ou PEG (polyéthylène glycol).

Le conteneur 16 comporte des tubulures obturées, par exemple par soudure. Ces tubulures ont été utilisées, avant leur obturation, pour introduire le plasma dans le conteneur 16.

Une étiquette adhésive est collée sur une face extérieure du colis 14. Cette étiquette adhésive comporte une puce de communication sans fil.

Chaque puce correspond à un identifiant unique.

Selon l'exemple proposé, la puce de communication sans fil est une puce RFID.

La puce RFID comporte une antenne radiofréquence, une mémoire et éventuellement un microprocesseur.

La mémoire de chaque puce comprend, par exemple, les informations suivantes : l'identifiant unique, un numéro de colis, un numéro de commande et/ou une donnée de contrôle.

L'appareil 12 est propre à mettre en œuvre un inventaire du contenu d'un réceptacle 10, c'est-à-dire des colis 14 et/ou des conteneurs 16 contenus dans chaque colis.

Le réceptacle 10 est, dans le mode de réalisation représenté sur la figure 1, une armoire à grille métallique.

Le réceptacle 10 comporte des roulettes 18 et des parois 20.

Les roulettes 18 permettent à un opérateur de pousser le réceptacle 10 pour la transporter d'un lieu à un autre. Le réceptacle est apte à être déplacé.

Selon l'exemple de la figure 1, le réceptacle comporte quatre roulettes 18 en contact avec le sol. Seules trois roulettes 18 sont visibles, la dernière roulette 18 formant un rectangle avec les roulettes 18 visibles.

La pluralité de parois 20 définit un volume intérieur V dans lequel les colis 14 sont destinés à se trouver.

Dans le cas particulier de la figure 1, le réceptacle 10 comporte au moins six parois 20, dont une paroi de support 22, une paroi supérieure 21 et trois parois transversales 24, et une double porte frontale 23.

Le réceptacle 10 forme alors une cage formée de grilles métalliques.

La double porte 23 est configurée pour s'ouvrir de sorte à permettre le remplissage du réceptacle 10 par des colis 14.

La paroi de support 22 est destinée à supporter les colis 14.

Chaque paroi 20 est formée d'une grille métallique.

Le réceptacle 10 comporte, en outre, une paroi intérieure et une pluralité de clayettes 25, par exemple dix clayettes, délimitant dans le volume intérieur des casiers, chaque casier étant destiné à recevoir un colis 14.

Le réceptacle 10 est vide lorsque le volume intérieur V est dépourvu de colis 14 alors que le réceptacle 10 est plein lorsque l'intégralité du volume intérieur V est remplie de colis 14.

Lorsque le réceptacle 10 est plein, le réceptacle 10 pèse un poids important, typiquement un poids supérieur à 200 kg. Il en résulte que le transport du réceptacle 10 plein est difficile pour un opérateur.

Alternativement, le réceptacle 10 comprend une palette formant une paroi de support et est déplacée à l'aide d'un transpalette ou d'un gerbeur, les roulettes du transpalette ou du gerbeur étant alors considérées comme les roulettes du réceptacle. Le nombre de colis 14 que la palette 10 est propre à accueillir est égal à 44 colis.

Alternativement, le réceptacle 10 est un caddie comportant cinq parois, dont une paroi de support et quatre parois transversales, et des roulettes. Chaque paroi est formée d'une grille métallique.

Le caddie a une hauteur inférieure ou égale à 1,20 mètre. Les conteneurs 16 sont placés directement dans le volume intérieur V défini par le caddie, sans être accueillis dans des colis.

L'appareil 12 comporte un portique 26 et au moins une borne de commande 27.

Le portique 26 comporte deux panneaux de lecture 28 reposant sur un sol 30.

Le sol 30 est le sol d'un emplacement où le portique est installé ou est formé par une partie inférieure du portique.

Il est défini une direction de traversée pour le portique 26. La direction de traversée est symbolisée par un axe X sur la figure 1.

Il est aussi défini une première direction transversale correspondant à la direction perpendiculaire à la partie inférieure 30. La première direction transversale est symbolisée par un axe Z sur la figure 1.

Une deuxième direction transversale est définie comme une direction perpendiculaire à la direction de traversée pour le portique 26 et perpendiculaire à la première direction transversale. La deuxième direction transversale est symbolisée par un axe Y sur la figure 1.

Dans le mode de réalisation représenté, le portique 26 comprend un toit 32. Le toit 32 comprend, par exemple, deux poutres s'étendant selon la deuxième direction transversale et reliant les deux panneaux de lecture 28.

Pour clarifier le propos, le premier panneau de lecture est référencé 28A dans la suite alors que le deuxième panneau de lecture est référencée 28B.

Le premier panneau de lecture 28A se présente sous la forme d'un parallélépipède.

Le premier panneau de lecture 28A présente une première dimension le long de la deuxième direction transversale Y, une deuxième dimension le long de la première direction transversale Z et une troisième dimension le long de la direction de traversée X.

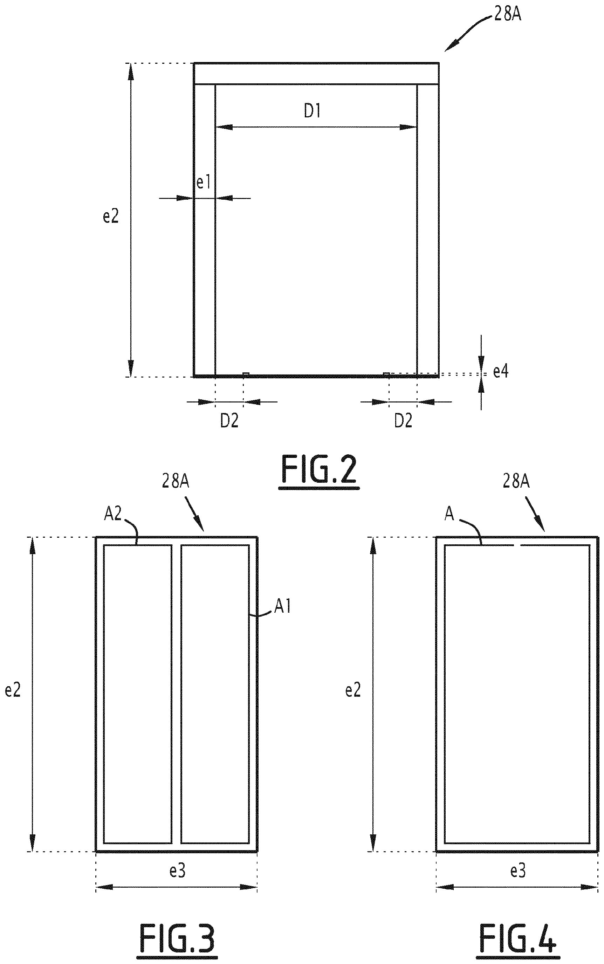

Comme visible sur la figure 2, la première dimension est notée e1. La première dimension e1 correspond à l'épaisseur.

La première dimension e1 est comprise entre 200 mm et 400 mm (millimètres).

Comme visible sur les figures 2 et 3, la deuxième dimension est notée e2. La deuxième dimension e2 correspond à la hauteur.

La deuxième dimension e2 est comprise 2000 mm et 3000 mm.

Comme visible sur la figure 3, la troisième dimension est notée e3. La troisième dimension e3 correspond à la profondeur.

La troisième dimension e3 est comprise entre 1000 mm et 2000 mm.

Le premier panneau de lecture 28A comporte une pluralité d'antennes A1 et A2.

Comme visible sur la figure 3, le premier panneau de lecture 28A comporte deux antennes A1 et A2.

Les antennes A1 et A2 sont placées l'une à côté de l'autre dans le premier panneau de lecture 28A.

Les antennes A1 et A2 sont espacées d'une distance comprise entre 50 mm et 150 mm.

Chaque antenne A1 et A2 est formée par une boucle rectangle présentant des coins arrondis et caractérisée par une hauteur, selon la première direction transversale, et une largeur, selon la direction de traversée.

Chaque antenne A1 et A2 présente une hauteur comprise entre 1500 mm et 2500 mm et une largeur comprise entre 400 mm et 600 mm.

Les antennes A1 et A2 sont de même largeur et longueur et sont superposables par translation suivant la direction de traversée X.

Chaque antenne présente un brin sortant et un brin entrant.

Chaque antenne A1, A2 comprend en outre une carte de commutation et d'accord.

D'une part, la carte est prévue pour la commutation, c'est-à-dire à utiliser deux relais mécaniques électromécaniques pour connecter et/ou déconnecter l'antenne. Ainsi, un relais est relié au brin sortant de l'antenne ou déconnecté, et l'autre relais est relié au brin entrant de l'antenne ou déconnecté.

D'autre part, la carte est prévue pour l'accord, c'est-à-dire à adapter l'impédance de l'antenne à environ (50+jo) ohms, jo étant une valeur complexe choisie pour respecter les conditions d'accord explicitées ci-après et à assurer un équilibrage.

Si l'antenne ne présence pas une impédance accordée, alors une partie de la puissance repart vers la source, qui est ici également le lecteur liés aux antennes. La puissance repartant vers la source est perdue et est susceptible de perturber le lecteur.

L'impédance souhaitée vérifie la condition suivante : Za=Zs* avec Za l'impédance de l'antenne et Zs* le conjugué complexe de l'impédance de la source. Ici, la source a une impédance égale à 50+0j ohms donc l'impédance souhaitée de l'antenne est 50+0j ohms et jo = 0j ohm.

L'équilibrage est la minimisation du courant en mode commun sur un câble coaxial reliant le lecteur et l'antenne.

La présence de métal, par exemple d'un réceptacle 10 comprenant une grille métallique, dans le champ de l'antenne impacte l'impédance de l'antenne donc les performances de l'antenne.

Plus l'impédance est proche de 50+0j ohms, meilleures sont les performances des panneaux de lecture. Une première des deux antennes est accordée en champ libre, c'est-à-dire en l'absence de métal entre les panneaux de lecture 28A, 28B, et la deuxième antenne est accordée à proximité de métal, par exemple de l'armoire ou du caddie à grille métallique. La présence de deux antennes permet ainsi d'obtenir des performances satisfaisantes en présence de réceptacle à grille métallique, mais également sans grille métallique. Les mêmes remarques que pour le premier panneau de lecture 28A s'appliquent pour le deuxième panneau de lecture 28B.

En particulier, le deuxième panneau de lecture 28B comporte deux antennes B1 et B2.

Les antennes A1, A2 du premier panneau de lecture 28A sont superposables avec les antennes B1, B2 du deuxième panneau de lecture 28B par translation suivant la deuxième direction de traversée Y.

Une antenne A1 et A2 du premier panneau de lecture 28A est associée de manière biunivoque à une antenne B1 et B2 du deuxième panneau de lecture 28B, pour former des paires d'antennes.

Ainsi, la première antenne A1 du premier panneau de lecture 28A et la première antenne B1 du deuxième panneau de lecture 28B sont associées pour former une première paire d'antennes tandis que la deuxième antenne A2 du premier panneau de lecture 28A et la deuxième antenne B2 du deuxième panneau de lecture 28B sont associées pour former une deuxième paire d'antennes.

Les paires d'antennes sont capables de lire des puces RFID, en particulier, les puces RFID circulant dans le portique 10.

De ce fait, les panneaux 28A et 28B sont qualifiés comme étant des « panneaux de lecture » de par leur capacité à lire des puces RFID.

Selon l'exemple de la figure 1, les deux panneaux de lecture 28A et 28B se font face.

Il est défini une première distance D1 comme étant la distance entre les deux panneaux de lecture 28A et 28B. La première distance D1 est la distance le long de la deuxième direction transversale Y.

La première distance D1 est supérieure ou égale à 1000 mm.

La première distance D1 est inférieure ou égale à 1 700 mm.

Selon un exemple particulier, le sol 30 comporte deux organes de guidage 34A et 34B.

Chaque organe de guidage 34A et 34B est positionné à une deuxième distance D2 d'un panneau de lecture respectif 28A et 28B. La deuxième distance D2 est la distance le long de la deuxième direction transversale Y.

La deuxième distance D2 a été choisie expérimentalement par le demandeur pour que les antennes A1, A2, B1 et B2 puissent lire les étiquettes RFID.

Aussi, la deuxième distance D2 est comprise entre 5 cm et 20 cm.

En variante, le déplacement du réceptacle 10 est mise en œuvre de sorte que la distance entre le réceptacle 10 et une des panneaux de lecture 28A, 28B soit inférieure à 10 cm. Dans ce contexte, la distance est mesurée comme la distance entre l'extrémité du réceptacle 10 la plus proche d'un panneau de lecture 28A ou 28B.

De préférence, la distance entre le réceptacle 10 et une des panneaux de lecture 28A, 28B est inférieure à 5 cm.

Chaque organe de guidage 34A et 34B se présente sous la forme d'un rail s'étendant le long de la direction de traversée X.

La coopération entre les roulettes 18 d'un réceptacle 10 manipulée par un opérateur et un des organes de guidage 34A et 34B assure que la distance entre les antennes radiofréquences des étiquettes et le panneau de lecture 34A et 34B correspondant soit inférieure à 10 cm.

Le portique 26 comprend, en outre, un indicateur 36. L'indicateur 36 indique si le portique 26 est apte à accueillir le passage d'un réceptacle.

L'indicateur 36 est, par exemple, un signal lumineux de couleur verte si la circulation d'un réceptacle dans le portique est autorisée, de couleur blanche si les panneaux de lecture 28A, 28B sont en cours de lecture de puces et rouge sinon.

La borne de commande 27 comprend un contrôleur 40 et une interface homme-machine 42.

Le contrôleur 40 est propre à commander chaque antenne A1, A2, B1 et B2.

En particulier, le contrôleur 40 est propre à injecter des courants contrôlés selon une loi de commande spécifique à chaque antenne A1, A2, B1 et B2.

Selon l'exemple présenté, le contrôleur 40 est propre à appliquer une première loi de commande des courants assurant que chaque paire d'antennes A1 et B1 d'une part et A2 et B2 d'autre part fonctionne alternativement en configuration dite de Helmholtz. Dans une telle première loi de commande, un même courant en phase est injecté dans chaque antenne A1 et B1 de la première paire d'antennes puis un autre courant de même valeur est injecté dans chaque antenne A2 et B2 de la deuxième paire d'antennes.

Chaque valeur de courant est maintenue pendant une période de temps dépendant d'un nombre d'étiquettes lues par les panneaux de lecture 28A, 28B.

L'interface homme-machine 42 est propre à afficher des informations.

L'interface homme-machine 42 comprend, par exemple, un écran 44 de chaque côté du portique 26 selon la direction de traversée X, les informations s'affichant sur les deux écrans ou sur un écran sélectionné. Ainsi, un opérateur est apte à obtenir les informations qu'il soit d'un côté ou de l'autre du portique.

L'interface homme-machine 42 est apte à afficher des informations générales sur la réception, des anomalies ou des informations de contrôle.

Selon le cas présenté, l'écran est tactile de sorte que l'opérateur est apte à interagir avec le portique, par exemple, en indiquant un numéro de commande ou en validant les informations affichées. L'écran tactile est compatible avec des gants.

Selon un autre exemple, l'écran présente des boutons.

Alternativement, l'interface homme-machine 42 ne comprend qu'un écran d'un seul côté du portique selon la direction de traversée X.

Le contrôleur 40 est, par exemple, intégré dans un des écrans 44 de l'interface homme-machine 42.

Le fonctionnement de l'appareil 10 est maintenant décrit en référence à un procédé de mise en œuvre d'un inventaire d'une pluralité de conteneurs 16.

Le procédé comprend une étape de fourniture de l'appareil 12 et une étape de fourniture d'un réceptacle 10 comprenant la pluralité de conteneurs 16 pour lequel un inventaire est à mettre en œuvre.

La pluralité de conteneurs 16 est répartie dans des colis 14, tel que décrit précédemment.

Le suivi est, par exemple, réalisé à l'échelle du colis.

Le procédé comporte également une étape de déplacement du réceptacle 10 pour traverser le portique 26 le long de la direction de traversée X.

Dans un mode de réalisation, au moins une roulette 18 du réceptacle 10 coopère avec un organe de guidage 34A, 34B pendant le déplacement.

L'étape de déplacement du réceptacle 10 est mise en œuvre alors que l'indicateur 36 autorise la circulation d'un réceptacle dans le portique.

Par exemple, selon un exemple particulier, une roulette 18 est mise en contact dans un organe de guidage 34A, 34B puis, l'opérateur pousse le réceptacle 10 de sorte que la roulette 18 reste en contact avec l'organe de guidage 34A, 34B tout au long du déplacement.

Selon un autre exemple, dans lequel le réceptacle comprend une palette déplacée à l'aide d'un transpalette ou d'un gerbeur, le transpalette est déplacé entre les organes de guidage 34A, 34B.

Le procédé comprend une étape de détection d'un passage d'un réceptacle 10 à l'aide d'au moins un capteur de détection, par exemple, des capteurs laser ou un capteur de masse. Le passage d'un réceptacle est détecté à chaque extrémité du portique 26 selon la direction de traversée X.

Lorsque le passage d'un réceptacle est détecté, alors une étape de lecture des puces de communication sans fil est mise en œuvre par au moins l'un des panneaux de lecture 28A, 28B lors du déplacement du réceptacle 10.

Par exemple, le contrôleur 40 met en œuvre la première loi de commande qui consiste à appliquer de manière alternative un courant sur la première paire d'antennes puis un courant sur la deuxième paire d'antennes.

Le signal lumineux de l'indicateur 36 devient alors blanc.

L'étape de lecture est effectuée dynamiquement ou statiquement, c'est-à-dire que le réceptacle se déplace ou est immobilisée entre les panneaux de lecture 28A, 28B pendant l'étape de lecture. Le réceptacle se déplace, par exemple, à moins de 5 km/h.

Il a été montré par le demandeur qu'une telle première loi de commande permet, malgré la présence des matériaux des colis 16 et du métal du réceptacle 10, de détecter toute puce de communication sans fil située entre les panneaux de lecture 28A, 28B.

Dans un mode de réalisation, l'étape de lecture est arrêtée après une durée déterminée et paramétrée par l'opérateur amenant le réceptacle ou préalablement. Le signal lumineux de l'indicateur 36 devient alors vert ou rouge selon le cas.

Si, pendant l'étape de lecture, aucune puce n'a été identifiée, alors l'interface homme-machine 42 propose, par exemple, de démarrer une nouvelle étape de lecture.

La borne de commande 27 a en mémoire la liste des puces détectées par le portique 26 et les colis et/ou les conteneurs correspondant grâce à l'identifiant unique respectif.

L'interface homme-machine 42 affiche des informations sur les étiquettes détectées, le suivi des conteneurs ou la réception.

L'interface homme-machine 42 affiche, par exemple, l'une ou plusieurs des informations suivantes : le nombre de puces détectées, les identifiants des puces détectées, les conteneurs correspondant et/ou la catégorie du produit contenu dans les conteneurs dans le colis, par exemple, la qualité du plasma.

Une étape de vérification est effectuée.

Pendant l'étape de vérification, la conformité du réceptacle 10 à réceptionner est vérifiée. Par exemple, si le nombre de colis est égal à un nombre attendu et si la nature du produit contenu dans les conteneurs correspond à celle à réceptionner, alors la réception est validée et un message confirmant la réception s'affiche sur l'interface homme-machine ; sinon, un message correspondant s'affiche sur l'interface homme-machine 42.

Si le nombre de colis est inférieur au nombre attendu, l'opérateur a, par exemple, le choix de valider le nombre, le reste des colis restant à réceptionner ultérieurement, de commencer une nouvelle étape de lecture ou de rentrer manuellement des puces qui n'auraient pas été détectées.

L'interface homme-machine 42 affiche éventuellement des informations supplémentaires. Les informations sont, par exemple, relatives à une anomalie ou un contrôle à effectuer.

Les informations sont, par exemple, l'une ou plusieurs parmi les suivantes :
- itinéraire passé et/ou prévu des conteneurs et/ou des colis,
- une erreur car les conteneurs des colis sont de plusieurs groupes qualité,
- une erreur liée à une différence entre le nombre de puces RFID détectées et le poids ou la contenance maximale du réceptacle,
- une erreur liée à une puce RFID inconnu, et/ou
- une erreur liée à une puce RFID détectée correspondant à un colis comprenant au moins un conteneur dont un suivi n'est pas disponible ou n'a pas été correctement effectué, par exemple, si un conteneur de plasma est périmé ou n'a pas été conservé à une température convenable.

Dans un mode de réalisation, le procédé comprend une étape de réconciliation, pendant laquelle les puces RFID détectées sont associées à un contenant dans lequel les conteneurs associés sont prévus d'être stockés. Ledit contenant est, par exemple, le réceptacle 10 si le réceptacle 10 est directement stocké.

L'interface homme-machine 42 affiche si le procédé comprend une telle étape de réconciliation.

Si au moins une des puces RFID détectées a déjà été associée à un autre contenant, alors un message d'erreur s'affiche sur l'interface homme-machine 42. L'interface homme-machine 42 est alors apte à indiquer le ou les colis concernés de sorte qu'un opérateur peut les retirer du réceptacle 10 et les mettre sur le contenant prévu. Alternativement, après une validation, ladite puce RFID sera associée avec le nouveau contenant.

Puis, la liste des puces RFID détectées est alors, par exemple, transmise à un système de gestion central d'un stock dont le portique gère les entrées et/ou les sorties. La transmission est, par exemple, réalisée par liaison Ethernet jusqu'à un serveur dédié.

Après transmission, la liste est supprimée du contrôleur 40 du portique.

Additionnellement ou alternativement, une étiquette adhésive, telle que décrite en regard des colis 14, est collée sur une face extérieure de chaque conteneur 16. Le portique 26 est alors prévu pour identifier les différents conteneurs 16, dont ceux présents dans un colis 14, sur le réceptacle 10.

Additionnellement, une étiquette adhésive, telle que décrite en regard des colis 14, est collée sur le réceptacle 10. Le portique 26 est alors prévu pour identifier le réceptacle 10.

Dans ces modes de réalisation, il est prévu un mode de fonctionnement du portique 26 dans lequel le portique 26 ne traite que les puces correspondant à un type d'objet, par exemple, les conteneurs 16, les colis 14 ou le réceptacle 10.

Le procédé de mise en œuvre d'un inventaire d'une pluralité de conteneurs de produits biologiques est ainsi de mise en œuvre plus rapide.

Plus généralement, cela permet de gagner du temps à la réception des colis 16.

Le sens de traversée du portique est indifférent, de sorte que l'opérateur peut choisir le sens le plus adapté à la situation. Typiquement, l'opérateur choisit le sens de traversée minimisant la distance à effectuer en poussant le réceptacle 10.

Cela assure également des contraintes moins pénibles pour l'opérateur.

Selon un mode de réalisation, le portique 26 comprend, en outre, un capteur prévu pour lire des codes-barres, le portique 26 étant apte à fonctionner dans un mode dégradé dans lequel un opérateur scanne avec ledit capteur un code-barres présent sur chaque étiquette de colis et/ou conteneur ou rentre grâce à l'interface homme-machine 24 des numéros respectifs correspondant aux codes-barres.

Selon un mode de réalisation, les étapes de déplacement et de lecture sont mises en œuvre dans une enceinte à atmosphère contrôlée.

Par exemple, l'enceinte est à une température contrôlée, typiquement comprise entre 0°C et 5°C, plus particulièrement égal à + 4°C.

Dans de tels cas, le gain en temps obtenu par l'emploi de l'appareil 12 permet, en outre, de mieux respecter les dispositions législatives visant à assurer une meilleure conservation des produits biologiques de chaque conteneur 16.

En variante, une autre loi de commande que la première loi de commande est utilisée.

Par exemple, au lieu d'appliquer dans chaque paire d'antennes un courant en phase, il est introduit un déphasage entre les deux courants.

Lorsque le déphasage est de 180°, il est obtenu une configuration dite de miroir magnétique permettant une lecture d'une puce RFID qui serait orientée de manière normale à la direction de traversée X.

Lorsque le déphasage est de 90°, il est obtenu une alternance entre la configuration dite de Helmoltz et la configuration dite de miroir magnétique. Cela permet notamment de lire les étiquettes, quelle que soit la direction des étiquettes.

Dans cette variante, le procédé de mise en œuvre d'un inventaire d'une pluralité de conteneurs de produits biologiques est également de mise en œuvre plus rapide que lors dans un inventaire par scan de code-barres.

A la figure 4, selon une alternative non couverte par les revendications, chaque panneau de lecture 28A, 28B comporte une unique antenne A, B.

Chaque antenne A, B est formée par une boucle. Chaque antenne A, B a, par exemple, la forme d'une boucle rectangle présentant des coins arrondis et caractérisée par une hauteur, selon la première direction transversale Z, et une largeur, selon la direction de traversée X.

Selon l'exemple présenté, la boucle présente une ouverture au milieu d'un côté supérieur selon sa largeur.

La hauteur est, par exemple, comprise entre 1500 mm et 2500 mm, et la largeur est, par exemple, comprise entre 750 mm et 1250 mm.

Les antennes A, B des deux panneaux de lecture 28A, 28B ont les mêmes dimensions et sont superposables par translation suivant la deuxième direction transversale Y.

Chaque antenne A, B comprend deux circuits d'accord commutés permettant de faire fonctionner chaque antenne sélectivement selon deux modes de fonctionnement différents : un mode « champ libre » et un mode « proximité métal », similairement aux deux antennes d'un même panneau de lecture dans le mode de réalisation précédent.

L'antenne A du premier panneau de lecture 28A est associée de manière biunivoque à l'antenne B du deuxième panneau de lecture 28B, pour former une paire d'antennes.

La paire d'antenne A, B est capable de lire des puces RFID, en particulier, les puces RFID circulant dans le portique 10, similairement au mode de réalisation décrit précédemment.

Le contrôleur 40 est propre à commander chaque antenne. En particulier, le contrôleur 40 est propre à injecter des courants contrôlés selon une loi de commande spécifique à chaque antenne.

Le procédé de mise en œuvre est similaire à ce qui a été décrit précédemment et les mêmes avantages sont obtenus.

## Revendications

1. Procédé de mise en œuvre d'un inventaire d'une pluralité de conteneurs (16) de produits biologiques, chaque conteneur (16) et/ou chaque colis (14) comprenant des conteneurs (16) étant muni d'une étiquette d'identification avec une puce de communication sans fil, le procédé comprenant les étapes de :
- fourniture d'un appareil (12) de mise en œuvre d'un inventaire, l'appareil (12) comportant un portique (26) définissant une direction de traversée (X), le portique comprenant deux panneaux de lecture (28A, 28B), les panneaux de lecture (28A, 28B) comportant chacun une pluralité d'antennes (A1, A2, B1, B2) adaptées pour lire une puce de communication sans fil placées l'une à côté de l'autre, une antenne (A1, A2, B1, B2) d'un panneau de lecture (28A, 28B) étant associée de manière biunivoque à une antenne (A1, A2, B1, B2) d'un autre panneau de lecture (28A, 28B) pour former des paires d'antennes (A1, B1 ; A2, B2),
- fourniture d'un réceptacle (10) apte à être déplacé, le réceptacle (10) portant la pluralité de conteneurs (16) pour lesquels un inventaire est à mettre en œuvre,
- déplacement du réceptacle (10) pour traverser le portique (26) le long de la direction de traversée, et
- lecture par les panneaux de lecture (28A, 28B) de la puce de communication sans fil de chaque conteneur et/ou colis (14, 16) lors du déplacement du réceptacle (10),
l'étape de lecture étant mise en œuvre par alimentation successive des paires d'antennes (A1, B1 ; A2, B2) en courant pendant une période de temps.

2. Procédé selon la revendication 1, dans lequel le portique (26) comporte un sol (30) comportant au moins deux organes de guidage (34A, 34B) s'étendant selon la direction de traversée, chaque organe de guidage (34A, 34B) étant associé à un panneau de lecture (28A, 28B) respectif et étant agencé à une distance inférieure à 20 centimètres (cm) d'un panneau de lecture (28A, 28B) respectif le long d'une direction transversale à la direction de traversée, chaque réceptacle (10) comprenant une pluralité de roulettes (18), au moins une roulette (18) étant prévue pour coopérer avec au moins un organe de guidage (34A, 34B).

3. Procédé selon la revendication 1 ou 2, dans lequel les étapes de déplacement et de lecture sont mises en œuvre dans une enceinte à atmosphère contrôlée.

4. Procédé selon l'une quelconque des revendications 1 à 3, dans lequel le réceptacle (10) comprend au moins une paroi formée d'une grille métallique.

5. Procédé selon l'une quelconque des revendications 1 à 4, dans lequel le courant appliqué dans chaque paire d'antennes (A1, B1 ; A2, B2) respecte l'une des conditions suivantes : être en phase, être déphasé de 90° et être déphasé de 180°.

6. Procédé selon l'une quelconque des revendications 1 à 5, dans lequel chaque panneau de lecture (28A, 28B) comporte uniquement deux antennes (A1, A2, B1, B2).

7. Appareil (12) de mise en œuvre d'un inventaire d'une pluralité de conteneurs (16) de produits biologiques, chaque conteneur (16) et/ou chaque colis (14) comprenant des conteneurs (16) étant muni d'une étiquette d'identification avec une puce de communication sans fil, la pluralité de conteneurs (16) pour lequel un inventaire est à mettre en œuvre faisant partie d'un réceptacle (10), l'appareil (12) comprenant:
- un portique (26) définissant une direction de traversée (X), le portique comprenant deux panneaux de lecture (28A, 28B) les panneaux de lecture (28A, 28B) comportant chacun une pluralité d'antennes (A1, A2, B1, B2) adaptées pour lire une puce de communication sans fil placées l'une à côté de l'autre, une antenne (A1, A2, B1, B2) d'un panneau de lecture (28A, 28B) étant associée de manière biunivoque à une antenne (A1, A2, B1, B2) d'un autre panneau de lecture (28A, 28B) pour former des paires d'antennes (A1, B1 ; A2, B2), et
- une borne de commande (27) comprenant un contrôleur (40) adapté pour commander les antennes (A1, A2, B1, B2 ; A, B) pour mettre en œuvre une étape lecture par les panneaux de lecture (28A, 28B) de la puce de communication sans fil de chaque conteneur et/ou colis (14, 16) lors du déplacement du réceptacle (10) pour traverser le portique (26) le long de la direction de traversée, l'étape de lecture étant mise en œuvre par alimentation successive des paires d'antennes (A1, A2, B1, B2) en courant pendant une période de temps.

## Patentansprüche

1. Verfahren zum Durchführen einer Bestandsaufnahme einer Vielzahl von Behältern (16) biologischer Produkte, wobei jeder Behälter (16) und/oder jedes Paket (14) mit Behältern (16) mit einem Identifikationsetikett mit einem Chip zur drahtlosen Kommunikation versehen ist, das Verfahren umfassend die folgenden Schritte:
- Bereitstellen einer Vorrichtung (12) zum Durchführen einer Bestandsaufnahme, die Vorrichtung (12) umfassend ein Portal (26), das eine Durchgangsrichtung (X) definiert, das Portal umfassend zwei Leseplatten (28A, 28B), die Leseplatten (28A, 28B) jeweils umfassend eine Vielzahl von Antennen (A1, A2, B1, B2), die angepasst sind, um einen drahtlosen Kommunikationschip zu lesen, die nebeneinander platziert sind, wobei eine Antenne (A1, A2, B1, B2) einer Leseplatte (28A, 28B) eineindeutig mit einer Antenne (A1, A2, B1, B2) einer anderen Leseplatte (28A, 28B) assoziiert ist, um Antennenpaare (A1, B1; A2, B2) zu bilden,
- Bereitstellen eines Behälters (10), der angepasst ist, um bewegt zu werden, wobei der Behälter (10) die Vielzahl von Behältern (16) trägt, für die eine Bestandsaufnahme durchgeführt werden soll,
- Bewegen des Behälters (10) zum Durchqueren des Portals (26) entlang der Durchquerungsrichtung, und
- Lesen des drahtlosen Kommunikationschips von jedem Behälter und/oder Paket (14, 16) durch die Leseplatten (28A, 28B) während der Bewegung des Behälters (10),
wobei der Leseschritt durch aufeinanderfolgendes Versorgen der Antennenpaare (A1, B1 A2, B2) mit Strom während einer Zeitperiode implementiert wird.

2. Verfahren nach Anspruch 1, wobei das Portal (26) einen Boden (30) umfasst, umfassend mindestens zwei Führungselemente (34A, 34B), die sich entlang der Durchquerungsrichtung erstrecken, wobei jedes Führungselement (34A, 34B) mit einer Leseplatte (28A, 28B) assoziiert ist und in einem Abstand von weniger als 20 Zentimetern (cm) von einer jeweiligen Leseplatte (28A, 28B) entlang einer Richtung quer zu der Durchquerungsrichtung angeordnet ist, jeder Behälter (10) umfassend eine Vielzahl von Rollen (18), wobei mindestens eine Rolle (18) bereitgestellt ist, um mit mindestens einem Führungselement (34A, 34B) zusammenzuwirken.

3. Verfahren nach Anspruch 1 oder 2, wobei die Schritte eines Bewegens und Lesens in einer Kammer mit kontrollierter Atmosphäre durchgeführt werden.

4. Verfahren nach einem der Ansprüche 1 bis 3, wobei der Behälter (10) mindestens eine Wand umfasst, die durch ein Metallgitter gebildet ist.

5. Verfahren nach einem der Ansprüche 1 bis 4, wobei der in jedem Antennenpaar (A1, B1; A2, B2) angelegte Strom eine der folgenden Bedingungen erfüllt: er ist gleichphasig, er ist um 90° phasenverschoben und er ist um 180° phasenverschoben.

6. Verfahren nach einem der Ansprüche 1 bis 5, wobei jede Leseplatte (28A, 28B) nur zwei Antennen (A1, A2, B1, B2) umfasst.

7. Vorrichtung (12) zum Durchführen einer Bestandsaufnahme einer Vielzahl von Behältern (16) biologischer Produkte, jeder Behälter (16) und/oder jedes Paket (14) mit Behältern (16) umfassend ein Identifikationsetikett, das mit einem drahtlosen Kommunikationschip versehen ist, wobei die Vielzahl von Behältern (16), für die eine Bestandsaufnahme durchgeführt werden soll, Teil eines Behälters (10) ist, die Vorrichtung (12) umfassend:
- ein Portal (26), das eine Durchgangsrichtung (X) definiert, das Portal umfassend zwei Leseplatten (28A, 28B), die Leseplatten (28A, 28B) jeweils umfassend eine Vielzahl von Antennen (A1, A2, B1, B2), die angepasst sind, um einen drahtlosen Kommunikationschip zu lesen, die nebeneinander platziert sind, wobei eine Antenne (A1, A2, B1, B2) einer Leseplatte (28A, 28B) eineindeutig mit einer Antenne (A1, A2, B1, B2) einer anderen Leseplatte (28A, 28B) assoziiert ist, um Antennenpaare (A1, B1; A2, B2) zu bilden, und
- einen Steueranschluss (27), umfassend eine Steuerung (40), die angepasst ist, um die Antennen (A1, A2, B1, B2; A, B) zu steuern, um einen Leseschritt durch die Leseplatten (28A, 28B) des drahtlosen Kommunikationschips von jedem Behälter und/oder Paket (14, 16) während der Bewegung des Behälters (10) zum Durchqueren des Portals (26) entlang der Durchquerungsrichtung durchzuführen, wobei der Leseschritt durch aufeinanderfolgendes Versorgen der Antennenpaare (A1, A2, B1, B2) mit Strom während einer Zeitperiode durchgeführt wird.

## Claims

1. A method of carrying out an inventory of a plurality of containers (16) of biological products, each container (16) and/or each package (14) comprising containers (16) being provided with an identification tag with a wireless communication chip, the method comprising the steps of:
- providing an apparatus (12) for carrying out an inventory, the apparatus (12) having a gantry (26) defining a traversing direction (X), the gantry comprising two reader panels (28A, 28B), the reader panels (28A, 28B) each having a plurality of antennas (A1, A2, B1, B2) adapted to read a wireless communication chip located adjacent to each other, an antenna (A1, A2, B1, B2) of a reader panel (28A, 28B) being associated with an antenna (A1, A2, B1, B2) of another reader panel (28A, 28B), to form antenna pairs (A1, B1; A2, B2),
- providing a receptacle (10) capable of being moved, the receptacle (10) carrying the plurality of containers (16) for which an inventory is to be carried out,
- moving the receptacle (10) through the gantry (26) along the traversing direction, and
- reading, by the reader panels (28A, 28B), the wireless communication chip of each container and/or package (14, 16) as the receptacle (10) moves,
the reading step being implemented by successively supplying the antenna pairs (A1, B1; A2, B2) with power for a period of time.

2. The method according to claim 1, wherein the gantry (26) comprises a floor (30) comprising at least two guide members (34A, 34B) extending along the traversing direction, each guide member (34A, 34B) being associated with a respective reader panel (28A, 28B) and being arranged at a distance of less than 20 centimetres (cm) away from a respective reader panel (28A, 28B) along a direction transverse to the traversing direction, each receptacle (10) comprising a plurality of castors (18), at least one castor (18) being adapted to cooperate with at least one guide member (34A, 34B).

3. The method according to claim 1 or 2, wherein the moving and reading steps are carried out in a controlled atmosphere chamber.

4. The method according to any one of claims 1 to 3, wherein the receptacle (10) comprises at least one wall formed of a metal grille.

5. The method according to any one of claims 1 to 4, wherein the current applied in each pair of antennas (A1, B1; A2, B2) complies with one of the following conditions: being in phase, being 90° out of phase and being 180° out of phase.

6. The method according to any one of claims 1 to 5, wherein each reader panel (28A, 28B) comprises only two antennas (A1, A2, B1, B2).

7. An apparatus (12) for carrying out an inventory of a plurality of containers (16) of biological products, each container (16) and/or each package (14) comprising containers (16) being provided with an identification tag with a wireless communication chip, the plurality of containers (16) for which an inventory is to be carried out being part of a receptacle (10), the apparatus (12) comprising:
- a gantry (26) defining a traversing direction (X), the gantry comprising two reader panels (28A, 28B), the reader panels (28A, 28B) each having a plurality of antennas (A1, A2, B1, B2) adapted to read a wireless communication chip located adjacent to each other, an antenna (A1, A2, B1, B2) of a reader panel (28A, 28B) being associated with an antenna (A1, A2, B1, B2) of another reader panel (28A, 28B), to form antenna pairs (A1, B1; A2, B2), and
- a control terminal (27) comprising a controller (40) adapted to control the antennas (A1, A2, B1, B2 ; A, B) to implement a step of reading, by the reader panels (28A, 28B), the wireless communication chip of each container and/or package (14, 16) as the receptacle (10) moves to traverse the gantry (26) along the traversing direction, the reading step being implemented by successively supplying power to the pairs of antennas (A1, A2, B1, B2) for a period of time.
